# EUROPEAN PATENT APPLICATION

(11) **EP 4 265 127 A1**
(43) Date of publication of application: **25.10.2023**
(21) Application number: 21906957.2
(22) Date of filing: 07.12.2021
(51) Int. Cl.: A23L 33/10, A23L 29/10, A23L 33/15, A61K 9/107

(54) **WATER-SOLUBLE EMULSION COMPOSITION CONTAINING NATURAL POLYPHENOL COMPOUND**

(30) Priority: 15.12.2020 KR 20200174987; 25.01.2021 KR 20210010126
(71) Applicant: Yun, Kwan Sik, Bucheon-si, Gyeonggi-do 14549 (KR)
(72) Inventor: Yun, Kwan Sik, Bucheon-si, Gyeonggi-do 14549 (KR)
(74) Representative: Modiano, Gabriella Diana
(86) International application number: PCT/KR2021/018428
(87) International publication number: WO 2022/131653

(57) **Abstract**

The present disclosure relates to a natural polyphenol compound-containing water-soluble emulsion composition containing a water-soluble antioxidant emulsion. A natural polyphenol compound-containing water-soluble emulsion composition according to one embodiment of the present disclosure may increase an absorption rate and efficacy of the material by dissolving a functional poorly water-soluble material, so that the functionality of the material may be used in various fields. In addition, since the composition contains a water-soluble antioxidant emulsion, it has excellent oxidation stability capable of preventing oxidation by oxygen in an aqueous solution.

## Description

### [Technical field]

The present disclosure relates to a natural polyphenol compound-containing water-soluble emulsion composition containing a water-soluble antioxidant emulsion.

### [Background Art]

Natural polyphenol compounds is a secondary metabolite which exist in a variety of plants and some microorganisms, and its representative functions can be largely divided into antioxidant activity and hormonal activity. Representative natural polyphenol compounds that are currently commercially used include catechins derived from green tea, and resveratrol, quercetin, and the like are known to have antioxidant activity. In addition, it has been reported that quercetin, a flavonol-based compound, has anticancer, anti-inflammatory, and diabetes preventive functions in addition to antioxidant effects.

Functional natural products including such natural polyphenol compound are mostly poorly water-soluble and have very low absorption in the body, resulting in low bioavailability. Therefore, there are limitations in the application of food, medicine, feed, and the like using these bioactive substances. In addition, existing water-soluble functional emulsions are O/W type emulsions, and are oxidized by oxygen contained in the aqueous phase and water, resulting in loss of antioxidant efficacy or rancidity.

On the other hand, as a representative natural antioxidant, α-Tocopherol is widely distributed in seed oil, grains, and green leafy vegetables, and as vitamin E, the α-Tocopherol is a major fat-soluble antioxidant present in cell membranes, and prevents oxidation of fats. Vitamin E may react directly with superoxide radicals (O₂·) as well as with a variety of oxygen radicals, including peroxy radicals (rOO·), CCl₃, and HO . Antioxidative activity is high in the order of δ > γ > β > α. As a mechanism of action, polyunsaturated fatty acids constituting cell membranes are easily oxidized by free radicals produced in cells, and tocopherol stops this oxidation process and removes free radicals to protect cell membranes from oxidative damage.

### [Disclosure]

### [Technical Problem]

The present disclosure is for the purpose of providing a natural polyphenol compound -containing water-soluble emulsion composition which may increase an absorption rate and efficacy of materials by dissolving a functional poorly soluble material in water and has excellent oxidation stability in an aqueous solution.

### [Technical Solution]

In order to achieve the above matter, the present disclosure provides a natural polyphenol compound-containing water-soluble emulsion composition containing a water-soluble antioxidant emulsion, including:
(1) the water-soluble antioxidant emulsion containing the following components to satisfy a total 100% by weight:
   1 to 40% by weight of tocopherol,
   1 to 40% by weight of catechin,
   1 to 30% by weight of a first surfactant having a hydrophilic-lipophilic balance (HLB) of 3 to 7,
   10 to 80% by weight of a second surfactant having a HLB of 10 to 14,
   10 to 45% by weight of a co-surfactant, and
   1 to 35% by weight of edible oil and fat; and
(2) a natural polyphenol compound emulsion containing the following components to satisfy a total 100% by weight:
   1 to 30% by weight of natural polyphenol compound,
   1 to 10% by weight of a first surfactant having a HLB of 3 to 7,
   1 to 20% by weight of a second surfactant having a HLB of 10 to 14, and
   40 to 97% by weight of a co-surfactant.

### [Advantageous Effects]

A natural polyphenol compound-containing water-soluble emulsion composition according to one embodiment of the present disclosure may increase an absorption rate and efficacy of the material by making a functional poorly water-soluble material water-soluble, so that the functionality of the material may be used in various fields.

In addition, since the composition contains a water-soluble antioxidant emulsion, it has excellent oxidation stability capable of preventing oxidation by oxygen in an aqueous solution.

### [Description of Drawings]

FIG. 1 is a photograph observed under an optical microscope of emulsified particles of a natural polyphenol compound-containing water-soluble emulsion composition of Comparative Example 33.
FIG. 2 is a photograph observed under an optical microscope of emulsified particles of a natural polyphenol compound-containing water-soluble emulsion composition of Example 1.

### [Modes of the Invention]

Hereinafter, a water-soluble antioxidant emulsion and a natural polyphenol compound emulsion according to one embodiment of the present disclosure will be described in detail.

The natural polyphenol compound-containing water-soluble emulsion composition according to an example embodiment of the present disclosure includes the water-soluble antioxidant emulsion and the natural polyphenol compound emulsion.

The water-soluble antioxidant emulsion includes 1 to 40% by weight of tocopherol, 1 to 40% by weight of catechin, 1 to 30% by weight of a first surfactant having a HLB of 3 to 7, 10 to 80% by weight of a second surfactant having a HLB of 10 to 14, 10 to 45% by weight of co-surfactant, and 1 to 35% by weight of edible oil and fat, to satisfy a total 100% by weight.

The tocopherol and catechin are included in the emulsion as fat-soluble and water-soluble antioxidants, respectively and a content can be appropriately set within the range of 1 to 40% by weight respectively. For example, the content may be 5 to 35% by weight, 5 to 25% by weight, 5 to 10% by weight, but not limited thereto. At this time, when contents of tocopherol and catechin are less than the above range, an antioxidant effect of addition is insignificant, and on the contrary, when the contents exceed the above range, there is a matter in that emulsion stability of the emulsion is significantly reduced.

The hydrophile-lipophile balance (HLB) is a value representing a degree of affinity of a surfactant for water and oil. The smaller the HLB value, the stronger the lipophilicity of the molecule as a whole, and the higher the HLB value, the stronger the hydrophilicity.

The water-soluble antioxidant emulsion and natural polyphenol compound emulsion include two types of surfactants with different HLB in terms of securing the emulsion stability of each emulsion and showing a surfactant effect while being well dissolved in a hydrophilic co-surfactant.

Using the first surfactant having a HLB of 3 to 7 and the second surfactant having a HLB of 10 to 14 prevents aggregation between emulsified particles, and is more effective in terms of maintaining respective antioxidant power of tocopherol and catechin which are antioxidants, in hydrophilic co-surfactant and edible fats and oils. When the HLB of the first surfactant is less than 3, an emulsion is not well formed, and when the HLB exceeds 7, aggregation between particles occurs, resulting in deterioration in emulsion stability. When the HLB of the second surfactant is less than 10, emulsification is difficult, and when the HLB exceeds 14, emulsion stability is deteriorated.

The first surfactant having a HLB of 3 to 7 may be used without limitation as long as it is known, and examples of the first surfactant may be one one selected from the group consisting of glycerin fatty acid ester, polyglycerin fatty acid ester, sorbitan fatty acid ester, lecithin, propylene glycol fatty acid ester, and a mixture thereof. In this case, a content of the first surfactant is 1 to 30% by weight based on a total weight of the water-soluble antioxidant emulsion. In one example, it may be selected from 5 to 20% by weight, 5 to 25% by weight, and 5 to 15% by weight. When the content of the first surfactant is less than the above range, emulsification action of the emulsified particles of edible oil and fat is weakened, making it difficult to form an emulsion, and when the content exceeds the above range, there is a matter in that emulsion stability is poor.

The second surfactant having a HLB of 10 to 14 may be one selected from the group consisting of polyglycerin fatty acid ester, polyoxyethylene sorbitan fatty acid ester, sucrose fatty acid ester, enzymatically degraded lecithin, and a mixture thereof, but is not limited thereto. At this time, the content of the second surfactant may be appropriately determined in the range of 10 to 80% by weight based on the total weight of the water-soluble antioxidant emulsion, for example, it may be 20 to 70% by weight, 30 to 60% by weight, 40 to 50% by weight, but is not limited thereto. When the content of the second surfactant satisfies the above numerical range, tocopherol and catechin may be stably dispersed in the water-soluble antioxidant emulsion without damaging the antioxidant ability of tocopherol and catechin.

The co-surfactant plays an auxiliary role in emulsification and is used as a dissolving agent. The co-surfactant may be one selected selected from the group consisting of glycerin, sorbitol, polyethylene glycol, propylene glycol, triacetin, and a mixture thereof, but is not limited thereto. The content of the co-surfactant may be 10 to 45% by weight based on a total weight of the water-soluble antioxidant emulsion. In one example, it may be selected from 10 to 40% by weight, 15 to 30% by weight, and 15 to 20% by weight. If the content of the co-surfactant is less than the above range, it is not easy to dissolve catechin, and when the content exceeds the above range, emulsion particles are not formed well and emulsion stability is deteriorated.

The edible oil and fat may be used without limitation as long as it is crude oil obtained from plants or animals or manufactured and processed using it as a raw material. For example, one or more oils and fats selected from soybean oil, corn oil, vegetable oil, high oleic vegetable oil, safflower oil, rice bran oil, palm oil, olive oil, sunflower oil, coconut oil, wheat germ oil, and medium-chain neutral fat (MCT oil) may be used. A content of the edible oil and fat may be 1 to 35% by weight based on a total weight of the composition and for example, it may be 1 to 30% by weight, 3 to 25% by weight, 5 to 20% by weight, but is not limited thereto. When the content is less than the above range, the amount of oil is too small to dissolve the tocopherol, and when the content exceeds the above range, the stability of the emulsion over time is poor.

The natural polyphenol compound emulsion includes 1 to 30% by weight of natural polyphenol compound, 1 to 10% by weight of a water-in-oil surfactant having a HLB of 3 to 7, 1 to 20% by weight of an oil-in-water surfactant of HLB of 10 to 14, and 40 to 97% by weight of a co-surfactant, to satisfy a total 100% by weight.

The natural polyphenol compound may be used without limitation as long as it has a biological effect on mammals when administered orally and not only glycosides but also nonglycosides are possible. Examples of the natural polyphenol compound include, but are in no way limited to, silymarin, curcumin, catechin, kaempferol, resveratrol, luteolin, quercetin, rutin, gallic acid, tannin, hesperetin, hesperidin, anthocyanidin, anthocyanin, naringenin, naringin, baicalein, and baicalin.

The natural polyphenol compound is included in an amount of 1 to 30% by weight based on a total weight of the natural polyphenol compound emulsion. For example, it may be 1 to 30% by weight, 3 to 25% by weight, 5 to 20% by weight, 10 to 20% by weight, but is not limited thereto. When a content of the natural polyphenol compound is less than the above range, an effect of addition is insignificant, and when the content exceeds the above range, it is difficult to form emulsified particles.

The natural polyphenol compound emulsion includes the first surfactant and the second surfactant having the HLB value described above in order to maintain the emulsion stability of the emulsion composition by exhibiting a surfactant effect while being well dissolved in the hydrophilic co-surfactant.

The first surfactant having a HLB of 3 to 7 is included in an amount of 1 to 10% by weight based on a total weight of the natural polyphenol compound emulsion. In one example, it may be selected from 3 to 10% by weight, 5 to 10% by weight, and 8 to 10% by weight. When the content of the first surfactant is less than the above range, emulsification action of the natural polyphenol compound emulsified particles is weakened, making it difficult to form an emulsion, and when the content exceeds the above range, there is a matter in that emulsion stability is lowered.

The second surfactant having a HLB of 10 to 14 is included in an amount of 1 to 20% by weight based on a total weight of the natural polyphenol compound emulsion. For example, it may be 3 to 20% by weight, 5 to 20% by weight, or 10 to 20% by weight, but is not limited thereto. At this time, when the content of the second surfactant is less than the above range, emulsification action on the hydrophilic co-surfactant is weakened, making it difficult to emulsify, and when the content exceeds 20% by weight, there is a matter in that it is difficult to maintain emulsion stability.

Specific types of the first surfactant and the second surfactant are as described above.

The co-surfactant is added to disperse and dissolve the natural polyphenol compound and play an auxiliary role in emulsification, and a content of the co-surfactant in the natural polyphenol compound emulsion is 40 to 97% by weight. For example, it may be 40 to 90% by weight, 40 to 80% by weight, 40 to 70% by weight, 50 to 60% by weight, but is not limited thereto. When the content of the co-surfactant is less than the above range, it is difficult to maintain emulsion stability, and when the content exceeds the above range, it is difficult to stably disperse the natural polyphenol compound.

The natural polyphenol compound-containing water-soluble emulsion composition includes 0.1 to 20% by weight of the water-soluble antioxidant emulsion and 80 to 99.9% by weight of the natural polyphenol compound emulsion. At this time, the water-soluble antioxidant emulsion may be 0.1 to 15% by weight, 0.1 to 10% by weight, 1 to 15% by weight, 1 to 10% by weight, but is not limited thereto. In addition, the natural polyphenol compound emulsion may be 80 to 99.9% by weight, 90 to 99.9% by weight, 90 to 99% by weight, but is not limited thereto.

The natural polyphenol compound-containing water-soluble emulsion composition of the present disclosure may increase an absorption rate and efficacy of the material by dissolving the natural polyphenol compound which is a functional poorly water-soluble material, so that the functionality of the material may be used in various fields. In addition, since the composition contains a water-soluble antioxidant emulsion, it has oxidation stability capable of preventing oxidation by oxygen in an aqueous solution.

### [Modes of the Invention]

Hereinafter, the present disclosure will be described in detail by way of Examples. However, these examples are not intended to limit the scope of the present disclosure.

### Examples

After preparing a water-soluble antioxidant emulsion and an natural polyphenol compound emulsion, respectively, with the compositions shown in Tables 1 to 13 below, a final natural polyphenol compound-containing water-soluble emulsion composition was prepared.

**[Table 1]**

| Components/Compoundi ng ratios (wt%) | | Prep. Ex. 1 | Prep. Ex. 2 | Prep. Ex. 3 | Prep. Ex. 4 | Prep. Ex. 5 | Prep. Ex. 6 | Prep. Ex. 7 | Prep. Ex. 8 |
|---|---|---|---|---|---|---|---|---|---|
| Tocopherol | | 10.0 | 10.0 | 15.0 | - | 10.0 | 10.0 | 10.0 | 10.0 |
| Catechin | | 5.0 | 5.0 | - | 15.0 | 5.0 | 5.0 | 5.0 | 5.0 |
| First surfactant | Glycerin fatty acid ester (HLB: 4) | 15.0 | - | 15.0 | 15.0 | 15.0 | - | - | 65.0 |
| | Sucrose fatty acid ester (HLB: 2) | - | 15.0 | - | - | - | - | - | - |
| | Polyglyceryl-6 diisostearate (HLB: 8) | | | | | | 15.0 | | |
| Second surfactant | Polyglycerin fatty acid ester (HLB: 12) | 50.0 | 50.0 | 50.0 | 50.0 | - | - | 65.0 | - |
| | Polyglyceryl-6 diisostearate (HLB: 8) | - | - | - | - | 50.0 | - | - | - |
| | PEG-18 Stearate (HLB: 18) | - | - | - | - | - | 50.0 | - | - |
| Glycerin | | 15.0 | 15.0 | 15.0 | 15.0 | 15.0 | 15.0 | 15.0 | 15.0 |
| Soybean oil | | 5.0 | 5.0 | 5.0 | 5.0 | 5.0 | 5.0 | 5.0 | 5.0 |

| | | | | | | | | | |
|---|---|---|---|---|---|---|---|---|---|
| Prep. Ex.: Preparation Example | | | | | | | | | |

After a first surfactant and a second surfactant were added to the homomixer, a mixture was stirred at 500 rpm or less while maintaining the temperature at 50 °C or more. Then, raw material 1 was prepared by sequentially adding catechin, soybean oil, and tocopherol.

Separately, raw material 2 was prepared by adding the second surfactant to glycerin.

At this time, 30% by weight and 70% by weight of a total amount of the second surfactant were used for the raw material 1 and the raw material 2, respectively.

While mixing the raw material 1 with the raw material 2, emulsification and dispersion was performed at 5,000 rpm or more using a homomixer to prepare a water-soluble antioxidant emulsion.

**[Table 2]**

| Components/Compounding ratios (wt%) | | Prep. Ex. 9 | Prep. Ex. 10 | Prep. Ex. 11 | Prep. Ex. 12 | Prep. Ex. 13 | Prep. Ex. 14 |
|---|---|---|---|---|---|---|---|
| Silymarin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 3]**

| Components/Compounding ratios (wt%) | | Prep. Ex. 15 | Prep. Ex. 16 | Prep. Ex. 17 | Prep. Ex. 18 | Prep. Ex. 19 | Prep. Ex. 20 |
|---|---|---|---|---|---|---|---|
| Curcumin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin | 20.0 | 20.0 | - | - | 30.0 | - |
| | fatty acid ester (HLB: 12) | | | | | | |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 4]**

| Components/Compounding ratios (wt%) | | Prep. Ex. 21 | Prep. Ex. 22 | Prep. Ex. 23 | Prep. Ex. 24 | Prep. Ex. 25 | Prep. Ex. 26 |
|---|---|---|---|---|---|---|---|
| Catechin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 5]**

| Components/Compounding ratios (wt%) | | Prep. Ex. 27 | Prep. Ex. 28 | Prep. Ex. 29 | Prep. Ex. 30 | Prep. Ex. 31 | Prep. Ex. 32 |
|---|---|---|---|---|---|---|---|
| Kaempferol | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 6]**

| Components/Compounding ratios (wt%) | | Prep. Ex. 33 | Prep. Ex. 34 | Prep. Ex. 35 | Prep. Ex. 36 | Prep. Ex. 37 | Prep. Ex. 38 |
|---|---|---|---|---|---|---|---|
| Resveratrol | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 7]**

| Components/Compounding ratios (wt%) | | Prep. Ex. 39 | Prep. Ex. 40 | Prep. Ex. 41 | Prep. Ex. 42 | Prep. Ex. 43 | Prep. Ex. 44 |
|---|---|---|---|---|---|---|---|
| Luteolin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 8]**

| Components/Compounding ratios (wt%) | | Prep. Ex. 45 | Prep. Ex. 46 | Prep. Ex. 47 | Prep. Ex. 48 | Prep. Ex. 49 | Prep. Ex. 50 |
|---|---|---|---|---|---|---|---|
| Quercetin | | 20.0 | 20.0 | 20.0 | 20.0 | 20.0 | 20.0 |
| First surfactant | Glycerin fatty | 10.0 | - | 10.0 | - | - | 30.0 |
| | acid ester (HLB:4) | | | | | | |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 9]**

| Components/Compounding ratios (wt%) | | Prep. Ex. 51 | Prep. Ex. 52 | Prep. Ex. 53 | Prep. Ex. 54 | Prep. Ex. 55 | Prep. Ex. 56 |
|---|---|---|---|---|---|---|---|
| Tannin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 10]**

| Components/Compounding ratios (wt%) | | Prep. Ex. 57 | Prep. Ex. 58 | Prep. Ex. 59 | Prep. Ex. 60 | Prep. Ex. 61 | Prep. Ex. 62 |
|---|---|---|---|---|---|---|---|
| Hesperidin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 11]**

| Components/Compounding ratios (wt%) | | Prep. Ex. 63 | Prep. Ex. 64 | Prep. Ex. 65 | Prep. Ex. 66 | Prep. Ex. 67 | Prep. Ex. 68 |
|---|---|---|---|---|---|---|---|
| Anthocyanidin | | 20.0 | 20.0 | 20.0 | 20.0 | 20.0 | 20.0 |
| First surfactant | Glycerin fatty | 10.0 | - | 10.0 | - | - | 30.0 |
| | acid ester (HLB:4) | | | | | | |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 12]**

| Components/Compounding ratios (wt%) | | Prep. Ex. 69 | Prep. Ex. 70 | Prep. Ex. 71 | Prep. Ex. 72 | Prep. Ex. 73 | Prep. Ex. 74 |
|---|---|---|---|---|---|---|---|
| Naringenin | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

**[Table 13]**

| Components/Compounding ratios (wt%) | | Prep. Ex. 75 | Prep. Ex. 76 | Prep. Ex. 77 | Prep. Ex. 78 | Prep. Ex. 79 | Prep. Ex. 80 |
|---|---|---|---|---|---|---|---|
| Baicalein | | 20.0 | 20.0 | 20.0 | 20.0 | 20.0 | 20.0 |
| First surfactant | Glycerin fatty acid ester (HLB:4) | 10.0 | - | 10.0 | - | - | 30.0 |
| | Sucrose fatty acid ester (HLB: 2) | - | 10.0 | - | - | - | - |
| | Polyglyceryl-6 diisostearate (HLB: 8) | | | - | 10.0 | | - |
| Second surfactant | Polyglycerin fatty acid ester (HLB: 12) | 20.0 | 20.0 | - | - | 30.0 | - |
| | Polyglyceryl-6 diisostearate (HLB: 8) | - | - | 20.0 | - | - | - |
| | PEG-18 Stearate (HLB: 18) | - | - | - | 20.0 | - | - |
| Glycerin | | 50.0 | 50.0 | 50.0 | 50.0 | 50.0 | 50.0 |

| | | | | | | | |
|---|---|---|---|---|---|---|---|
| Prep. Ex.: Preparation Example | | | | | | | |

After a first surfactant and a second surfactant were added to the homomixer, a mixture was stirred at 500 rpm or less while maintaining the temperature at 50 °C or more. Then, when a natural polyphenol compound raw material was added and the natural polyphenol compound began to get wet in the emulsion, stirring of the homomixer was started, and emulsification and dispersion were performed at 5,000 rpm or more while gradually increasing the rpm. When the emulsification was completed, glycerin was added and stirred at 500 rpm or less to prepare a natural polyphenol compound emulsion.

**[Table 14]**

| Classification (wt%) | Ex. 1 | Ex. 2 | Comp. Ex. 1 | Comp. Ex. 2 | Comp. Ex. 3 | Comp. Ex. 4 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Silymarin emulsion | Prep. Ex. 9 (99 wt%) | Prep. Ex. 9 (90 wt%) | Prep. Ex. 9 (99 wt%) | Prep. Ex. 9 (90 wt%) | Prep. Ex. 9 (99 wt%) | Prep. Ex. 9 (90 wt%) |

| | | | | | | |
|---|---|---|---|---|---|---|
| Ex.: Example Comp. Ex.: Comparative Example Prep. Ex.: Preparation Example | | | | | | |

**[Table 15]**

| Classification (wt%) | Ex. 3 | Ex. 4 | Comp. Ex. 5 | Comp. Ex. 6 | Comp. Ex. 7 | Comp. Ex. 8 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Curcumin emulsion | Prep. Ex. 15 (99 wt%) | Prep. Ex. 15 (90 wt%) | Prep. Ex. 15 (99 wt%) | Prep. Ex. 15 (90 wt%) | Prep. Ex. 15 (99 wt%) | Prep. Ex. 15 (90 wt%) |

| | | | | | | |
|---|---|---|---|---|---|---|
| Ex.: Example Comp. Ex.: Comparative Example Prep. Ex.: Preparation Example | | | | | | |

**[Table 16]**

| Classification (wt%) | Ex. 5 | Ex. 6 | Comp. Ex. 9 | Comp. Ex. 10 | Comp. Ex. 11 | Comp. Ex. 12 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Catechin emulsion | Prep. Ex. 21 (99 wt%) | Prep. Ex. 21 (90 wt%) | Prep. Ex. 21 (99 wt%) | Prep. Ex. 21 (90 wt%) | Prep. Ex. 21 (99 wt%) | Prep. Ex. 21 (90 wt%) |

| | | | | | | |
|---|---|---|---|---|---|---|
| Ex.: Example Comp. Ex.: Comparative Example Prep. Ex.: Preparation Example | | | | | | |

**[Table 17]**

| Classification (wt%) | Ex. 7 | Ex. 8 | Comp. Ex. 13 | Comp. Ex. 14 | Comp. Ex. 15 | Comp. Ex. 16 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Kaempferol emulsion | Prep. Ex. 27 (99 wt%) | Prep. Ex. 27 (90 wt%) | Prep. Ex. 27 (99 wt%) | Prep. Ex. 27 (90 wt%) | Prep. Ex. 27 (99 wt%) | Prep. Ex. 27 (90 wt%) |

| | | | | | | |
|---|---|---|---|---|---|---|
| Ex.: Example Comp. Ex.: Comparative Example Prep. Ex.: Preparation Example | | | | | | |

**[Table 18]**

| Classification (wt%) | Ex. 9 | Ex. 10 | Comp. Ex. 17 | Comp. Ex. 18 | Comp. Ex. 19 | Comp. Ex. 20 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Resveratrol emulsion | Prep. Ex. 33 (99 wt%) | Prep. Ex. 33 (90 wt%) | Prep. Ex. 33 (99 wt%) | Prep. Ex. 33 (90 wt%) | Prep. Ex. 33 (99 wt%) | Prep. Ex. 33 (90 wt%) |

| | | | | | | |
|---|---|---|---|---|---|---|
| Ex.: Example Comp. Ex.: Comparative Example Prep. Ex.: Preparation Example | | | | | | |

**[Table 19]**

| Classification (wt%) | Ex. 11 | Ex. 12 | Comp. Ex. 21 | Comp. Ex. 22 | Comp. Ex. 23 | Comp. Ex. 24 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Luteolin emulsion | Prep. Ex. 39 (99 wt%) | Prep. Ex. 39 (90 wt%) | Prep. Ex. 39 (99 wt%) | Prep. Ex. 39 (90 wt%) | Prep. Ex. 39 (99 wt%) | Prep. Ex. 39 (90 wt%) |

| | | | | | | |
|---|---|---|---|---|---|---|
| Ex.: Example Comp. Ex.: Comparative Example Prep. Ex.: Preparation Example | | | | | | |

**[Table 20]**

| Classification (wt%) | Ex. 13 | Ex. 14 | Comp. Ex. 25 | Comp. Ex. 26 | Comp. Ex. 27 | Comp. Ex. 28 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Quercetin emulsion | Prep. Ex. 45 (99 wt%) | Prep. Ex. 45 (90 wt%) | Prep. Ex. 45 (99 wt%) | Prep. Ex. 45 (90 wt%) | Prep. Ex. 45 (99 wt%) | Prep. Ex. 45 (90 wt%) |

| | | | | | | |
|---|---|---|---|---|---|---|
| Ex.: Example Comp. Ex.: Comparative Example Prep. Ex.: Preparation Example | | | | | | |

**[Table 21]**

| Classification (wt%) | Ex. 15 | Ex. 16 | Comp. Ex. 29 | Comp. Ex. 30 | Comp. Ex. 31 | Comp. Ex. 32 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Tannin emulsion | Prep. Ex. 51 (99 wt%) | Prep. Ex. 51 (90 wt%) | Prep. Ex. 51 (99 wt%) | Prep. Ex. 51 (90 wt%) | Prep. Ex. 51 (99 wt%) | Prep. Ex. 51 (90 wt%) |

| | | | | | | |
|---|---|---|---|---|---|---|
| Ex.: Example Comp. Ex.: Comparative Example Prep. Ex.: Preparation Example | | | | | | |

**[Table 22]**

| Classification (wt%) | Ex. 17 | Ex. 18 | Comp. Ex. 33 | Comp. Ex. 34 | Comp. Ex. 35 | Comp. Ex. 36 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Hesperidin emulsion | Prep. Ex. 57 (99 wt%) | Prep. Ex. 57 (90 wt%) | Prep. Ex. 57 (99 wt%) | Prep. Ex. 57 (90 wt%) | Prep. Ex. 57 (99 wt%) | Prep. Ex. 57 (90 wt%) |

| | | | | | | |
|---|---|---|---|---|---|---|
| Ex.: Example Comp. Ex.: Comparative Example Prep. Ex.: Preparation Example | | | | | | |

**[Table 23]**

| Classification (wt%) | Ex. 19 | Ex. 20 | Comp. Ex. 37 | Comp. Ex. 38 | Comp. Ex. 39 | Comp. Ex. 40 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Anthocyanidin emulsion | Prep. Ex. 63 (99 wt%) | Prep. Ex. 63 (90 wt%) | Prep. Ex. 63 (99 wt%) | Prep. Ex. 63 (90 wt%) | Prep. Ex. 63 (99 wt%) | Prep. Ex. 63 (90 wt%) |

| | | | | | | |
|---|---|---|---|---|---|---|
| Ex.: Example Comp. Ex.: Comparative Example Prep. Ex.: Preparation Example | | | | | | |

**[Table 24]**

| Classification (wt%) | Ex. 21 | Ex. 22 | Comp. Ex. 41 | Comp. Ex. 42 | Comp. Ex.43 | Comp. Ex. 44 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Naringenin emulsion | Prep. Ex. 69 (99 wt%) | Prep. Ex. 69 (90 wt%) | Prep. Ex. 51 (99 wt%) | Prep. Ex. 69 (90 wt%) | Prep. Ex. 69 (99 wt%) | Prep. Ex. 69 (90 wt%) |

| | | | | | | |
|---|---|---|---|---|---|---|
| Ex.: Example Comp. Ex.: Comparative Example Prep. Ex.: Preparation Example | | | | | | |

**[Table 25]**

| Classification (wt%) | Ex. 23 | Ex. 24 | Comp. Ex. 45 | Comp. Ex. 46 | Comp. Ex. 47 | Comp. Ex. 48 |
|---|---|---|---|---|---|---|
| Water-soluble antioxidant emulsion | Prep. Ex. 1 (1 wt%) | Prep. Ex. 1 (10 wt%) | Prep. Ex. 3 (1 wt%) | Prep. Ex. 3 (10 wt%) | Prep. Ex. 4 (1 wt%) | Prep. Ex. 4 (10 wt%) |
| Baicalein emulsion | Prep. Ex. 75 (99 wt%) | Prep. Ex. 75 (90 wt%) | Prep. Ex. 75 (99 wt%) | Prep. Ex. 75 (90 wt%) | Prep. Ex. 75 (99 wt%) | Prep. Ex. 75 (90 wt%) |

| | | | | | | |
|---|---|---|---|---|---|---|
| Ex.: Example Comp. Ex.: Comparative Example Prep. Ex.: Preparation Example | | | | | | |

According to the composition described in Tables 14 to 25, the water-soluble antioxidant emulsion prepared above was added to the natural polyphenol compound emulsion thus obtained, and mixing and dispersing were performed at 500 rpm or less while checking the properties of the emulsion to prepare an natural polyphenol compound-containing water-soluble emulsion composition containing a water-soluble antioxidant emulsion.

### Experimental Example 1: Antioxidant effect measurement

An antioxidant effect of the natural polyphenol compound-containing water-soluble emulsion composition including the water-soluble antioxidant emulsion was measured using a DPPH measurement method.

DPPH radical scavenging activity measurement is the most commonly used method for measuring antioxidant activity and for evaluating antioxidant activity by color change, when a sample with excellent antioxidant activity is treated, the color change is clear and when a sample with low effect is treated, there is not much change in purple color, so that the degree of this change is measured by measuring absorbance using a spectrophotometer (517 nm) to compare the antioxidant activity, and the higher the value, the higher the antioxidant effect.

The experimental conditions (harsh condition test: oxidation induction by leaving at 40 °C for 1 week) are as follows.
② Solvent - Ethanol
② Content - 0.01 wt% dispersion, 0.1 wt% dispersion
③ Method - Using a shaking waterbath for 2 hours at 30°C.
④ Filter - Using 0.45µm cartridge filter after natural filtration using a No.5 filter paper

Experimental results are shown in Tables 18 to 25 below.

**[Table 26]**

| Classification | Prep. Ex. 1 | Ex. 1 | Ex. 2 | Comp. Ex. 1 | Comp. Ex. 2 | Comp. Ex. 3 | Comp. Ex. 4 |
|---|---|---|---|---|---|---|---|
| 0.01 wt% | 83.1 | 54.8 | 61.9 | 43.8 | 52.3 | 41.2 | 48.9 |
| 0.1 wt% | 94.3 | 65.8 | 73.5 | 58.6 | 64.7 | 54.1 | 62.5 |

| | | | | | | | |
|---|---|---|---|---|---|---|---|
| Prep. Ex.: Preparation Example Ex.: Example Comp. Ex.: Comparative Example | | | | | | | |

**[Table 27]**

| Classification | Ex. 3 | Ex. 4 | Comp. Ex. 5 | Comp. Ex. 6 | Comp. Ex. 7 | Comp. Ex. 8 |
|---|---|---|---|---|---|---|
| 0.01 wt% | 60.2 | 69.1 | 53.4 | 59.2 | 49.5 | 55.6 |
| 0.1 wt% | 72.3 | 80.8 | 66.7 | 70.7 | 60.7 | 68.1 |

| | | | | | | |
|---|---|---|---|---|---|---|
| Ex.: Example Comp. Ex.: Comparative Example | | | | | | |

**[Table 28]**

| Classification | Ex. 5 | Ex. 6 | Comp. Ex. 9 | Comp. Ex. 10 | Comp. Ex. 11 | Comp. Ex. 12 |
|---|---|---|---|---|---|---|
| 0.01 wt% | 74.3 | 79.1 | 71.2 | 73.9 | 68.5 | 69.8 |
| 0.1 wt% | 85.5 | 88.2 | 80.8 | 83.1 | 76.6 | 79.4 |

| | | | | | | |
|---|---|---|---|---|---|---|
| Ex.: Example Comp. Ex.: Comparative Example | | | | | | |

**[Table 29]**

| Classification | Ex. 7 | Ex. 8 | Comp. Ex. 13 | Comp. Ex. 14 | Comp. Ex. 15 | Comp. Ex. 16 |
|---|---|---|---|---|---|---|
| 0.01 wt% | 69.2 | 77.5 | 65.1 | 68.8 | 60.7 | 66.5 |
| 0.1 wt% | 78.6 | 82.4 | 72.5 | 75.5 | 66.3 | 75.1 |

| | | | | | | |
|---|---|---|---|---|---|---|
| Ex.: Example Comp. Ex.: Comparative Example | | | | | | |

**[Table 30]**

| Classification | Ex. 9 | Ex. 10 | Comp. Ex. 17 | Comp. Ex. 18 | Comp. Ex. 19 | Comp. Ex. 20 |
|---|---|---|---|---|---|---|
| 0.01 wt% | 68.8 | 78.2 | 66.5 | 67.3 | 61.2 | 69.9 |
| 0.1 wt% | 77.7 | 88.2 | 71.8 | 75.6 | 69.2 | 73.2 |

| | | | | | | |
|---|---|---|---|---|---|---|
| Ex.: Example Comp. Ex.: Comparative Example | | | | | | |

**[Table 31]**

| Classification | Ex. 11 | Ex. 12 | Comp. Ex. 21 | Comp. Ex. 22 | Comp. Ex. 23 | Comp. Ex. 24 |
|---|---|---|---|---|---|---|
| 0.01 wt% | 70.8 | 79.2 | 65.1 | 68.5 | 63.3 | 68.7 |
| 0.\0 wt% | 81.4 | 87.4 | 77.8 | 79.7 | 70.6 | 80.2 |

| | | | | | | |
|---|---|---|---|---|---|---|
| Ex.: Example Comp. Ex.: Comparative Example | | | | | | |

**[Table 32]**

| Classification | Ex. 13 | Ex. 14 | Comp. Ex. 25 | Comp. Ex. 26 | Comp. Ex. 27 | Comp. Ex. 28 |
|---|---|---|---|---|---|---|
| 0.01 wt% | 77.5 | 82.5 | 73.9 | 75.6 | 70.1 | 72.3 |
| 0.1 wt% | 85.6 | 91.1 | 80.7 | 84.3 | 78.2 | 79.8 |

| | | | | | | |
|---|---|---|---|---|---|---|
| Ex.: Example Comp. Ex.: Comparative Example | | | | | | |

**[Table 33]**

| Classification | Ex. 15 | Ex. 16 | Comp. Ex. 29 | Comp. Ex. 30 | Comp. Ex. 31 | Comp. Ex. 32 |
|---|---|---|---|---|---|---|
| 0.01 wt% | 72.5 | 77.2 | 68.4 | 71.2 | 65.3 | 68.5 |
| 0.1 wt% | 79.8 | 85.6 | 76.4 | 78.2 | 71.1 | 77.3 |

| | | | | | | |
|---|---|---|---|---|---|---|
| Ex.: Example Comp. Ex.: Comparative Example | | | | | | |

**[Table 34]**

| Classification | Ex. 17 | Ex. 18 | Comp. Ex. 33 | Comp. Ex. 34 | Comp. Ex. 35 | Comp. Ex. 36 |
|---|---|---|---|---|---|---|
| 0.01 wt% | 75.5 | 79.2 | 70.8 | 72.1 | 68.8 | 69.2 |
| 0.\0 wt% | 81.9 | 87.2 | 75.4 | 79.6 | 73.2 | 74.2 |

| | | | | | | |
|---|---|---|---|---|---|---|
| Ex.: Example Comp. Ex.: Comparative Example | | | | | | |

**[Table 35]**

| Classification | Ex. 19 | Ex. 20 | Comp. Ex. 37 | Comp. Ex. 38 | Comp. Ex. 39 | Comp. Ex. 40 |
|---|---|---|---|---|---|---|
| 0.01 wt% | 74.4 | 78.8 | 67.8 | 70.1 | 66.2 | 67.5 |
| 0.1 wt% | 80.9 | 85.6 | 76.2 | 78.2 | 71.2 | 76.2 |

| | | | | | | |
|---|---|---|---|---|---|---|
| Ex.: Example Comp. Ex.: Comparative Example | | | | | | |

**[Table 36]**

| Classification | Ex. 21 | Ex. 22 | Comp. Ex. 41 | Comp. Ex. 42 | Comp. Ex. 43 | Comp. Ex. 44 |
|---|---|---|---|---|---|---|
| 0.01 wt% | 72.5 | 79.1 | 69.5 | 71.2 | 68.2 | 73.2 |
| 0.1 wt% | 79.6 | 83.3 | 74.1 | 77.1 | 74.1 | 74.6 |

| | | | | | | |
|---|---|---|---|---|---|---|
| Ex.: Example Comp. Ex.: Comparative Example | | | | | | |

**[Table 37]**

| Classification | Ex. 23 | Ex. 24 | Comp. Ex. 45 | Comp. Ex. 46 | Comp. Ex. 47 | Comp. Ex. 48 |
|---|---|---|---|---|---|---|
| 0.01 wt% | 75.2 | 79.9 | 68.3 | 72.8 | 69.1 | 70.5 |
| 0.1 wt% | 80.4 | 85.5 | 74.6 | 77.1 | 76.1 | 79.3 |

| | | | | | | |
|---|---|---|---|---|---|---|
| Ex.: Example Comp. Ex.: Comparative Example | | | | | | |

As shown in Tables 26 to 37, when using a water-soluble antioxidant emulsion of Preparation Examples 3 and 4 that do not contain catechin or tocopherol in the water-soluble antioxidant emulsion, it was confirmed that the antioxidant effect of the natural polyphenol compound-containing water-soluble emulsion composition is low.

### Experimental Example 2: Water solubility confirmation

According to the composition shown in Tables 38 to 61, the water-soluble antioxidant emulsion was added to the natural polyphenol compound emulsion, and then mixing and dispersing were performed at 500 rpm or less while checking the properties of the emulsion to prepare an natural polyphenol compound-containing water-soluble emulsion composition containing a water-soluble antioxidant emulsion.

Water solubility of the prepared natural polyphenol compound-containing water-soluble emulsion composition was confirmed as follows. 50 ml of distilled water was maintained at a water temperature of 38 °C in a constant temperature water bath, and 1 g of the emulsion compositions of Examples and Comparative Examples was added while maintaining a speed of 100 rpm. After 1 hour of centrifugation and filtering, an organic material content was quantified by measuring a water content using a Karl Fisher method.

**[Table 38]**

| Classificati on (wt%) | Ex. 1 | Comp. Ex. 33 | Comp. Ex. 34 | Comp. Ex. 35 | Comp. Ex. 36 | Comp. Ex. 37 | Comp. Ex. 38 | Comp. Ex. 39 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Silymarin emulsion | Prep. Ex. 9 (99 wt%) | Prep. Ex. 10 (99 wt%) | Prep. Ex. 11 (99 wt%) | Prep. Ex. 12 (99 wt%) | Prep. Ex. 13 (99 wt%) | Prep. Ex. 14 (99 wt%) | Prep. Ex. 9 (99 wt%) | Prep. Ex. 9 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 39]**

| Classification (wt%) | Comp. Ex. 40 | Comp. Ex. 41 | Comp. Ex. 42 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Silymarin emulsion | Prep. Ex. 9 (99 wt%) | Prep. Ex. 9 (99 wt%) | Prep. Ex. 9 (99 wt%) |

| | | | |
|---|---|---|---|
| Comp. Ex.: Comparative Example Prep. Ex.: Preparation Example | | | |

**[Table 40]**

| Classification (wt%) | Ex. 3 | Comp. Ex. 43 | Comp. Ex. 44 | Comp. Ex. 45 | Comp. Ex. 46 | Comp. Ex. 47 | Comp. Ex. 48 | Comp. Ex. 49 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Curcumin emulsion | Prep. Ex. 15 (99 wt%) | Prep. Ex. 16 (99 wt%) | Prep. Ex. 17 (99 wt%) | Prep. Ex. 18 (99 wt%) | Prep. Ex. 19 (99 wt%) | Prep. Ex. 20 (99 wt%) | Prep. Ex. 15 (99 wt%) | Prep. Ex. 15 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 41]**

| Classification (wt%) | Comp. Ex. 50 | Comp. Ex. 51 | Comp. Ex. 52 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Curcumin emulsion | Prep. Ex. 15 (99 wt%) | Prep. Ex. 15 (99 wt%) | Prep. Ex. 15 (99 wt%) |

| | | | |
|---|---|---|---|
| Comp. Ex.: Comparative Example Prep. Ex.: Preparation Example | | | |

**[Table 42]**

| Classification (wt%) | Ex. 5 | Comp. Ex. 53 | Comp. Ex. 54 | Comp. Ex. 55 | Comp. Ex. 56 | Comp. Ex. 57 | Comp. Ex. 58 | Comp. Ex. 59 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Catechin emulsion | Prep. Ex. 21 (99 wt%) | Prep. Ex. 22 (99 wt%) | Prep. Ex. 23 (99 wt%) | Prep. Ex. 24 (99 wt%) | Prep. Ex. 25 (99 wt%) | Prep. Ex. 26 (99 wt%) | Prep. Ex. 21 (99 wt%) | Prep. Ex. 21 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 43]**

| Classification (wt%) | Comp. Ex. 60 | Comp. Ex. 61 | Comp. Ex. 62 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Catechin emulsion | Prep. Ex. 21 (99 wt%) | Prep. Ex. 21 (99 wt%) | Prep. Ex. 21 (99 wt%) |

| | | | |
|---|---|---|---|
| Comp. Ex.: Comparative Example Prep. Ex.: Preparation Example | | | |

**[Table 44]**

| Classification (wt%) | Ex. 7 | Comp. Ex. 63 | Comp. Ex. 64 | Comp. Ex. 65 | Comp. Ex. 66 | Comp. Ex. 67 | Comp. Ex. 68 | Comp. Ex. 69 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Kaempferol emulsion | Prep. Ex. 27 (99 wt%) | Prep. Ex. 28 (99 wt%) | Prep. Ex. 29 (99 wt%) | Prep. Ex. 30 (99 wt%) | Prep. Ex. 31 (99 wt%) | Prep. Ex. 32 (99 wt%) | Prep. Ex. 27 (99 wt%) | Prep. Ex. 27 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 45]**

| Classification (wt%) | Comp. Ex. 70 | Comp. Ex. 71 | Comp. Ex. 72 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Kaempferol emulsion | Prep. Ex. 27 (99 wt%) | Prep. Ex. 27 (99 wt%) | Prep. Ex. 27 (99 wt%) |

| | | | |
|---|---|---|---|
| Comp. Ex.: Comparative Example Prep. Ex.: Preparation Example | | | |

**[Table 46]**

| Classification (wt%) | Ex. 9 | Comp. Ex. 73 | Comp. Ex. 74 | Comp. Ex. 75 | Comp. Ex. 76 | Comp. Ex. 77 | Comp. Ex. 78 | Comp. Ex. 79 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Resveratrol emulsion | Prep. Ex. 33 (99 wt%) | Prep. Ex. 34 (99 wt%) | Prep. Ex. 35 (99 wt%) | Prep. Ex. 36 (99 wt%) | Prep. Ex. 37 (99 wt%) | Prep. Ex. 38 (99 wt%) | Prep. Ex. 33 (99 wt%) | Prep. Ex. 33 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 47]**

| Classification (wt%) | Comp. Ex. 80 | Comp. Ex. 81 | Comp. Ex. 82 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Resveratrol Aemulsion | Prep. Ex. 33 (99 wt%) | Prep. Ex. 33 (99 wt%) | Prep. Ex. 33 (99 wt%) |

| | | | |
|---|---|---|---|
| Comp. Ex.: Comparative Example | | | |

**[Table 48]**

| Classification (wt%) | Ex. 11 | Comp. Ex. 83 | Comp. Ex. 84 | Comp. Ex. 85 | Comp. Ex. 86 | Comp. Ex. 87 | Comp. Ex. 88 | Comp. Ex. 89 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Luteolin emulsion | Prep. Ex. 39 (99 wt%) | Prep. Ex. 40 (99 wt%) | Prep. Ex. 41 (99 wt%) | Prep. Ex. 42 (99 wt%) | Prep. Ex. 43 (99 wt%) | Prep. Ex. 44 (99 wt%) | Prep. Ex. 39 (99 wt%) | Prep. Ex. 39 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 49]**

| Classification (wt%) | Comp. Ex. 90 | Comp. Ex. 91 | Comp. Ex. 92 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Luteolin emulsion | Prep. Ex. 39 (99 wt%) | Prep. Ex. 9 (99 wt%) | Prep. Ex. 39 (99 wt%) |

| | | | |
|---|---|---|---|
| Comp. Ex.: Comparative Example Prep. Ex.: Preparation Example | | | |

**[Table 50]**

| Classification (wt%) | Ex. 13 | Comp. Ex. 93 | Comp. Ex. 94 | Comp. Ex. 95 | Comp. Ex. 96 | Comp. Ex. 97 | Comp. Ex. 98 | Comp. Ex. 99 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Quercetin emulsion | Prep. Ex. 45 (99 wt%) | Prep. Ex. 46 (99 wt%) | Prep. Ex. 47 (99 wt%) | Prep. Ex. 48 (99 wt%) | Prep. Ex. 49 (99 wt%) | Prep. Ex. 50 (99 wt%) | Prep. Ex. 45 (99 wt%) | Prep. Ex. 45 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 51]**

| Classification (wt%) | Comp. Ex. 100 | Comp. Ex. 101 | Comp. Ex. 102 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Quercetin emulsion | Prep. Ex. 45 (99 wt%) | Prep. Ex. 45 (99 wt%) | Prep. Ex. 45 (99 wt%) |

| | | | |
|---|---|---|---|
| Comp. Ex.: Comparative Example Prep. Ex.: Preparation Example | | | |

**[Table 52]**

| Classification (wt%) | Ex. 15 | Comp. Ex. 103 | Comp. Ex. 104 | Comp. Ex. 105 | Comp. Ex. 106 | Comp. Ex. 107 | Comp. Ex. 108 | Comp. Ex. 109 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Tannin emulsion | Prep. Ex. 51 (99 wt%) | Prep. Ex. 52 (99 wt%) | Prep. Ex. 53 (99 wt%) | Prep. Ex. 54 (99 wt%) | Prep. Ex. 55 (99 wt%) | Prep. Ex. 56 (99 wt%) | Prep. Ex. 51 (99 wt%) | Prep. Ex. 51 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 53]**

| Classification (wt%) | Comp. Ex. 110 | Comp. Ex. 111 | Comp. Ex. 112 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Tannin emulsion | Prep. Ex. 51 (99 wt%) | Prep. Ex. 51 (99 wt%) | Prep. Ex. 51 (99 wt%) |

| | | | |
|---|---|---|---|
| Comp. Ex.: Comparative Example Prep. Ex.: Preparation Example | | | |

**[Table 54]**

| Classification (wt%) | Ex. 17 | Comp. Ex. 113 | Comp. Ex. 114 | Comp. Ex. 115 | Comp. Ex. 116 | Comp. Ex. 117 | Comp. Ex. 118 | Comp. Ex. 119 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Hesperidin emulsion | Prep. Ex. 57 (99 wt%) | Prep. Ex. 58 (99 wt%) | Prep. Ex. 59 (99 wt%) | Prep. Ex. 60 (99 wt%) | Prep. Ex. 61 (99 wt%) | Prep. Ex. 62 (99 wt%) | Prep. Ex. 57 (99 wt%) | Prep. Ex. 57 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 55]**

| Classification (wt%) | Comp. Ex. 120 | Comp. Ex. 121 | Comp. Ex. 122 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Hesperidin emulsion | Prep. Ex. 57 (99 wt%) | Prep. Ex. 57 (99 wt%) | Prep. Ex. 57 (99 wt%) |

| | | | |
|---|---|---|---|
| Comp. Ex.: Comparative Example Prep. Ex.: Preparation Example | | | |

**[Table 56]**

| Classification (wt%) | Ex. 19 | Comp. Ex. 123 | Comp. Ex. 124 | Comp. Ex. 125 | Comp. Ex. 126 | Comp. Ex. 127 | Comp. Ex. 128 | Comp. Ex. 129 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Anthocyanidin emulsion | Prep. Ex. 63 (99 wt%) | Prep. Ex. 64 (99 wt%) | Prep. Ex. 65 (99 wt%) | Prep. Ex. 66 (99 wt%) | Prep. Ex. 67 (99 wt%) | Prep. Ex. 68 (99 wt%) | Prep. Ex. 63 (99 wt%) | Prep. Ex. 63 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 57]**

| Classification (wt%) | Comp. Ex. 130 | Comp. Ex. 131 | Comp. Ex. 132 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Anthocyanidin emulsion | Prep. Ex. 63 (99 wt%) | Prep. Ex. 63 (99 wt%) | Prep. Ex. 63 (99 wt%) |

| | | | |
|---|---|---|---|
| Comp. Ex.: Comparative Example Prep. Ex.: Preparation Example | | | |

**[Table 58]**

| Classification (wt%) | Ex. 21 | Comp. Ex. 123 | Comp. Ex. 134 | Comp. Ex. 135 | Comp. Ex. 136 | Comp. Ex. 137 | Comp. Ex. 138 | Comp. Ex. 139 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Naringenin emulsion | Prep. Ex. 63 (99 wt%) | Prep. Ex. 64 (99 wt%) | Prep. Ex. 65 (99 wt%) | Prep. Ex. 66 (99 wt%) | Prep. Ex. 67 (99 wt%) | Prep. Ex. 68 (99 wt%) | Prep. Ex. 63 (99 wt%) | Prep. Ex. 63 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 59]**

| Classification (wt%) | Comp. Ex. 140 | Comp. Ex. 141 | Comp. Ex. 142 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Naringenin emulsion | Prep. Ex. 63 (99 wt%) | Prep. Ex. 63 (99 wt%) | Prep. Ex. 63 (99 wt%) |

| | | | |
|---|---|---|---|
| Comp. Ex.: Comparative Example Prep. Ex.: Preparation Example | | | |

**[Table 60]**

| Classification (wt%) | Ex. 23 | Comp. Ex. 143 | Comp. Ex. 144 | Comp. Ex. 145 | Comp. Ex. 146 | Comp. Ex. 147 | Comp. Ex. 148 | Comp. Ex. 149 |
|---|---|---|---|---|---|---|---|---|
| Water-soluble antioxidant emulsion | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 1 (1 wt%) | Prep. Ex. 2 (1 wt%) | Prep. Ex. 5 (1 wt%) |
| Baicalein emulsion | Prep. Ex. 63 (99 wt%) | Prep. Ex. 64 (99 wt%) | Prep. Ex. 65 (99 wt%) | Prep. Ex. 66 (99 wt%) | Prep. Ex. 67 (99 wt%) | Prep. Ex. 68 (99 wt%) | Prep. Ex. 63 (99 wt%) | Prep. Ex. 63 (99 wt%) |

| | | | | | | | | |
|---|---|---|---|---|---|---|---|---|
| Ex.: Example Comp. Ex.: Comparative Example Prep. Ex.: Preparation Example | | | | | | | | |

**[Table 61]**

| Classification (wt%) | Comp. Ex. 150 | Comp. Ex. 151 | Comp. Ex. 152 |
|---|---|---|---|
| Water-soluble antioxidant emulsion | Prep. Ex. 6 (1 wt%) | Prep. Ex. 7 (1 wt%) | Prep. Ex. 8 (1 wt%) |
| Baicalein emulsion | Prep. Ex. 63 (99 wt%) | Prep. Ex. 63 (99 wt%) | Prep. Ex. 63 (99 wt%) |

| | | | |
|---|---|---|---|
| Comp. Ex.: Comparative Example Prep. Ex.: Preparation Example | | | |

**[Table 62]**

| Classificati on | Ex. 1 | Comp. Ex. 33 | Comp. Ex. 34 | Comp. Ex. 35 | Comp. Ex. 36 | Comp. Ex. 37 | Comp. Ex. 38 | Comp. Ex. 39 | Comp. Ex. 40 | Comp. Ex. 41 | Comp. Ex. 42 |
|---|---|---|---|---|---|---|---|---|---|---|---|
| Water solubility | 98.1% | 28.9% | 21.8% | 28.6% | 32.6% | 18.6% | 15.7% | 22.6% | 25.4% | 18..8% | 32.7% |

| | | | | | | | | | | | |
|---|---|---|---|---|---|---|---|---|---|---|---|
| Ex.: Example Comp. Ex.: Comparative Example | | | | | | | | | | | |

**[Table 63]**

| Classifica tion | Generi c Powder ed Silyma rin | Generi c Powder ed Curcu min | Generi c Powder ed Catechi n | Generi c Powder ed kaempf erol | Generi c Powder ed Resver atrol | Generi c Powder ed Luteoli n | Generi c powder ed Querce tin | Generi c Powder ed Tannin | Generi c powder ed Hesper idin | Generi c Powder ed Anthoc yanidin | Generi c Powder ed Naring enin | Generi c Powder ed Baicale in |
|---|---|---|---|---|---|---|---|---|---|---|---|---|
| Water solubility | 0.00% | 0.00% | 0.22% | 0.01% | 0.00% | 0.02% | 0.00% | 15.2% | 0.00% | 0.02% | 0.04% | 0.00% |

| | | | | | | | | | | | | |
|---|---|---|---|---|---|---|---|---|---|---|---|---|
| Comp. Ex.: Comparative Example | | | | | | | | | | | | |

**[Table 64]**

| Classificati on | Ex. 3 | Comp. Ex. 43 | Comp. Ex. 44 | Comp. Ex. 45 | Comp. Ex. 46 | Comp. Ex. 47 | Comp. Ex. 48 | Comp. Ex. 49 | Comp. Ex. 50 | Comp. Ex. 51 | Comp. Ex. 52 |
|---|---|---|---|---|---|---|---|---|---|---|---|
| Water solubility | 98.2% | 22.8% | 28.6% | 19.3% | 29.1% | 17.7% | 21.4% | 22.8% | 29.3% | 31.1% | 28.6% |

| | | | | | | | | | | | |
|---|---|---|---|---|---|---|---|---|---|---|---|
| Ex.: Example Comp. Ex.: Comparative Example | | | | | | | | | | | |

**[Table 65]**

| Classifica tion | Ex. 5 | Comp. Ex. 103 | Comp. Ex. 104 | Comp. Ex. 105 | Comp. Ex. 106 | Comp. Ex. 107 | Comp. Ex. 108 | Comp. Ex. 109 | Comp. Ex. 110 | Comp. Ex. 111 | Comp. Ex. 112 |
|---|---|---|---|---|---|---|---|---|---|---|---|
| Water solubility | 99.4% | 15.3% | 25.3% | 24.1% | 22.2% | 19.6% | 17.5% | 18.5% | 25.3% | 21.4% | 12.8% |

| | | | | | | | | | | | |
|---|---|---|---|---|---|---|---|---|---|---|---|
| Ex.: Example Comp. Ex.: Comparative Example | | | | | | | | | | | |

**[Table 66]**

| Classification | Ex. 7 | Comp. Ex. 63 | Comp. Ex. 64 | Comp. Ex. 65 | Comp. Ex. 66 | Comp. Ex. 67 | Comp. Ex. 68 | Comp. Ex. 69 | Comp. Ex. 70 | Comp. Ex. 71 | Comp. Ex. 72 |
|---|---|---|---|---|---|---|---|---|---|---|---|
| Water solubility | 98.5 % | 30.2% | 25.2% | 18.4% | 14.4% | 19.6% | 22.8% | 24.7% | 32.2% | 30.1% | 27.5% |

| | | | | | | | | | | | |
|---|---|---|---|---|---|---|---|---|---|---|---|
| Ex.: Example Comp. Ex.: Comparative Example | | | | | | | | | | | |

**[Table 67]**

| Classification | Ex. 9 | Comp. Ex. 73 | Comp. Ex. 74 | Comp. Ex. 75 | Comp. Ex. 76 | Comp. Ex. 77 | Comp. Ex. 78 | Comp. Ex. 79 | Comp. Ex. 80 | Comp. Ex. 81 | Comp. Ex. 82 |
|---|---|---|---|---|---|---|---|---|---|---|---|
| Water solubility | 98.9% | 31.2% | 28.5% | 21.2% | 18.6% | 23.8% | 21.1% | 28.5% | 16.8% | 27.6% | 24.5% |

| | | | | | | | | | | | |
|---|---|---|---|---|---|---|---|---|---|---|---|
| Ex.: Example Comp. Ex.: Comparative Example | | | | | | | | | | | |

**[Table 68]**

| Classification | Ex. 11 | Comp. Ex. 83 | Comp. Ex. 84 | Comp. Ex. 85 | Comp. Ex. 86 | Comp. Ex. 87 | Comp. Ex. 88 | Comp. Ex. 89 | Comp. Ex. 90 | Comp. Ex. 91 | Comp. Ex. 92 |
|---|---|---|---|---|---|---|---|---|---|---|---|
| Water solubility | 98.7% | 19.6% | 18.8% | 24.5% | 23.4% | 28.8% | 20.4% | 27.1% | 28.8% | 26.1% | 19.9% |

| | | | | | | | | | | | |
|---|---|---|---|---|---|---|---|---|---|---|---|
| Ex.: Example Comp. Ex.: Comparative Example | | | | | | | | | | | |

**[Table 69]**

| Classification | Ex. 13 | Comp. Ex. 93 | Comp. Ex. 94 | Comp. Ex. 95 | Comp. Ex. 96 | Comp. Ex. 97 | Comp. Ex. 98 | Comp. Ex. 99 | Comp. Ex. 100 | Comp. Ex. 101 | Comp. Ex. 102 |
|---|---|---|---|---|---|---|---|---|---|---|---|
| Water solubility | 98.8% | 23.5% | 22.4% | 29.3% | 20.4% | 20.6% | 18.3% | 22.9% | 30.1% | 28.1% | 23.7% |

| | | | | | | | | | | | |
|---|---|---|---|---|---|---|---|---|---|---|---|
| Ex.: Example Comp. Ex.: Comparative Example | | | | | | | | | | | |

**[Table 70]**

| Classification | Ex. 15 | Comp. Ex. 103 | Comp. Ex. 104 | Comp. Ex. 105 | Comp. Ex. 106 | Comp. Ex. 107 | Comp. Ex. 108 | Comp. Ex. 109 | Comp. Ex. 110 | Comp. Ex. 111 | Comp. Ex. 112 |
|---|---|---|---|---|---|---|---|---|---|---|---|
| Water solubility | 99.8% | 18.1% | 20.8% | 19.6% | 28.1% | 25.5% | 21.6% | 24.4% | 23.5% | 25.5% | 28.1% |

| | | | | | | | | | | | |
|---|---|---|---|---|---|---|---|---|---|---|---|
| Ex.: Example Comp. Ex.: Comparative Example | | | | | | | | | | | |

**[Table 71]**

| Classifica tion | Ex. 17 | Comp. Ex. 113 | Comp. Ex. 114 | Comp. Ex. 115 | Comp. Ex. 116 | Comp. Ex. 117 | Comp. Ex. 118 | Comp. Ex. 119 | Comp. Ex. 120 | Comp. Ex. 121 | Comp. Ex. 122 |
|---|---|---|---|---|---|---|---|---|---|---|---|
| Water solubility | 97.5% | 26.6% | 24.2% | 28.9% | 27.3% | 18.0% | 17.5% | 21.3% | 24.1% | 22.0% | 29.1% |

| | | | | | | | | | | | |
|---|---|---|---|---|---|---|---|---|---|---|---|
| Ex.: Example Comp. Ex.: Comparative Example | | | | | | | | | | | |

**[Table 72]**

| Classification | Ex. 19 | Comp. Ex. 123 | Comp. Ex. 124 | Comp. Ex. 125 | Comp. Ex. 126 | Comp. Ex. 127 | Comp. Ex. 128 | Comp. Ex. 129 | Comp. Ex. 130 | Comp. Ex. 131 | Comp. Ex. 132 |
|---|---|---|---|---|---|---|---|---|---|---|---|
| Water solubility | 99.3 % | 23.0% | 24.5% | 22.8% | 29.8% | 31.4% | 32.1% | 28.1% | 21.0% | 23.1% | 22.5% |

| | | | | | | | | | | | |
|---|---|---|---|---|---|---|---|---|---|---|---|
| Ex.: Example Comp. Ex.: Comparative Example | | | | | | | | | | | |

**[Table 73]**

| Classification | Ex. 21 | Comp. Ex. 133 | Comp. Ex. 134 | Comp. Ex. 135 | Comp. Ex. 136 | Comp. Ex. 137 | Comp. Ex. 138 | Comp. Ex. 139 | Comp. Ex. 140 | Comp. Ex. 141 | Comp. Ex. 142 |
|---|---|---|---|---|---|---|---|---|---|---|---|
| Water solubility | 97.0% | 26.5% | 24.4% | 21.3% | 28.7% | 15.6% | 17.9% | 18.8% | 23.3% | 22.8% | 21.9% |

| | | | | | | | | | | | |
|---|---|---|---|---|---|---|---|---|---|---|---|
| Ex.: Example Comp. Ex.: Comparative Example | | | | | | | | | | | |

**[Table 74]**

| Classification | Ex. 23 | Comp. Ex. 143 | Comp. Ex. 144 | Comp. Ex. 145 | Comp. Ex. 146 | Comp. Ex. 147 | Comp. Ex. 148 | Comp. Ex. 149 | Comp. Ex. 150 | Comp. Ex. 151 | Comp. Ex. 152 |
|---|---|---|---|---|---|---|---|---|---|---|---|
| Water solubility | 96.8% | 29.1% | 27.5% | 22.1% | 26.3% | 25.7% | 20.4% | 25.1% | 22.8% | 27.6% | 28.8% |

| | | | | | | | | | | | |
|---|---|---|---|---|---|---|---|---|---|---|---|
| Ex.: Example Comp. Ex.: Comparative Example | | | | | | | | | | | |

As shown in Tables 62 to 74, it was found that the compositions of Examples 1 to 15 using the water-soluble antioxidant emulsion and the natural polyphenol compound emulsion containing two kinds of surfactants having different hydrophile-lipophile balance (HLB) have very high water solubility compared to the emulsions of the comparative examples.

### Experimental Example 3: Confirmation of water-dispersible emulsified particles

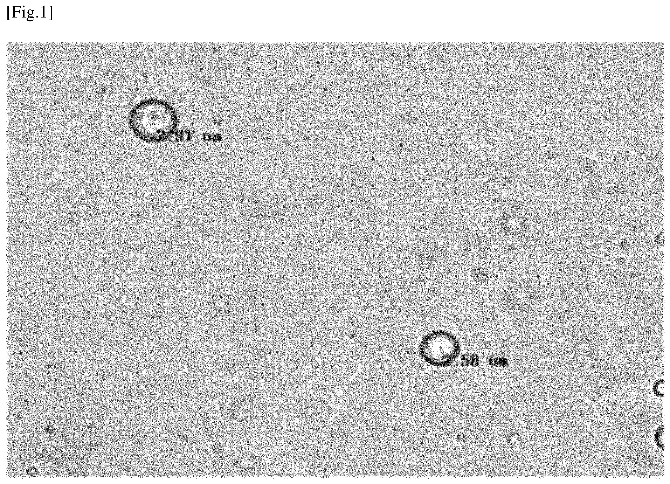

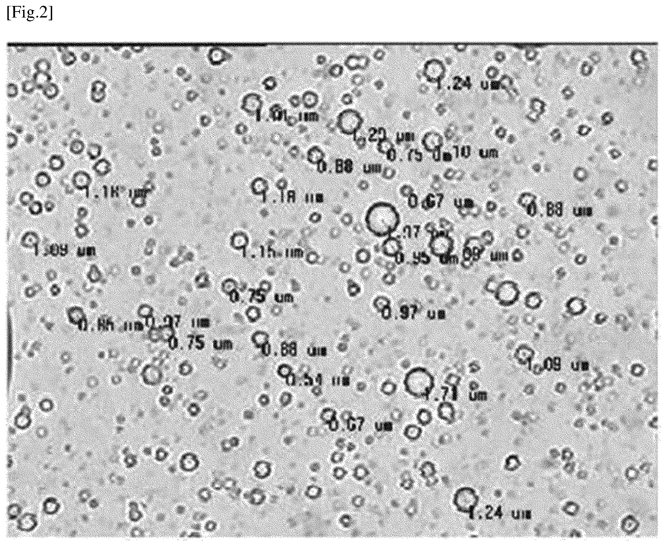

An emulsified particle size of the natural polyphenol compound-containing water-soluble emulsion composition prepared according to Tables 38 to 61 was confirmed as follows. 50 ml of distilled water was maintained at a water temperature of 38 °C in a constant temperature water bath, and 1 g of the emulsion compositions of Examples and Comparative Examples was added while maintaining a speed of 100 rpm. After 1 hour, emulsified particles were observed using an optical microscope, and the results are shown in FIGS. 1 and 2.

The size of emulsified particles in the distilled water was measured using a Zetasizer Nano ZS (Malvern Instrument, U.K.). At this time, the sample was repeatedly measured three times, and an average value is shown in Table 75 below.

**[Table 75]**

| Classification | Ex. 1 | Ex. 3 | Ex. 5 | Ex. 7 | Ex. 9 | Ex. 11 | Ex. 13 | Ex. 15 | Ex. 17 | Ex. 19 | Ex. 21 | Ex. 23 |
|---|---|---|---|---|---|---|---|---|---|---|---|---|
| Emulsion particle size (µm) | 1.13 | 1.24 | 1.20 | 1.52 | 1.75 | 1.56 | 1.12 | 1.98 | 1.55 | 1.22 | 1.34 | 1.46 |

| | | | | | | | | | | | | |
|---|---|---|---|---|---|---|---|---|---|---|---|---|
| Ex.: Example | | | | | | | | | | | | |

Referring to Table 75 and FIGS. 1 and 2, the natural polyphenol compound-containing water-soluble emulsion composition corresponding to one embodiment of the present disclosure forms emulsified particles of 1.0 to 2.5 µm, whereas the natural polyphenol compound-containing water-soluble emulsion composition of Comparative Examples 33 to 152 did not form emulsion particles, so that the size of emulsion particles could not be measured. From this, it can be expected that the natural polyphenol compound-containing water-soluble emulsion composition according to the present disclosure may be absorbed in the living body and exhibit high bioavailability.

## Claims

1. A natural polyphenol compound-containing water-soluble emulsion composition containing a water-soluble antioxidant emulsion, comprising:
(1) the water-soluble antioxidant emulsion containing the following components to satisfy a total 100% by weight:
1 to 40% by weight of tocopherol,
1 to 40% by weight of catechin,
1 to 30% by weight of a first surfactant having a hydrophilic-lipophilic balance (HLB) of 3 to 7,
10 to 80% by weight of a second surfactant having a HLB of 10 to 14,
10 to 45% by weight of a co-surfactant, and
1 to 35% by weight of edible oil and fat; and
(2) a natural polyphenol compound emulsion containing the following components to satisfy a total 100% by weight:
1 to 30% by weight of natural polyphenol compound,
1 to 10% by weight of a first surfactant having a HLB of 3 to 7,
1 to 20% by weight of a second surfactant having a HLB of 10 to 14, and
40 to 97% by weight of a co-surfactant.

2. The water-soluble emulsion composition of claim 1, wherein the natural polyphenol compound-containing water-soluble emulsion composition includes 0.1 to 20% by weight of the water-soluble antioxidant emulsion and 80 to 99.9% by weight of the natural polyphenol compound emulsion.

3. The water-soluble emulsion composition of claim 1 or 2, wherein the natural polyphenol compound is one selected from the group consisting of silymarin, curcumin, catechin, kaempferol, resveratrol, luteolin, quercetin, rutin, gallic acid, tannin, hesperetin, hesperidin, anthocyanidin, anthocyanin, naringenin, naringin, baicalein, baicalin, and a mixture thereof.

4. The water-soluble emulsion composition of claim 1 or 2, wherein the first surfactant is one selected from the group consisting of glycerin fatty acid ester, polyglycerin fatty acid ester, sorbitan fatty acid ester, lecithin, propylene glycol fatty acid ester, and a mixture thereof.

5. The water-soluble emulsion composition of claim 1 or 2, wherein the second surfactant is one selected from the group consisting of polyglycerin fatty acid ester, polyoxyethylene sorbitan fatty acid ester, sucrose fatty acid ester, enzymatically degraded lecithin, and a mixture thereof.

6. The water-soluble emulsion composition of claim 1 or 2, wherein the co-surfactant is one selected from the group consisting of glycerin, sorbitol, polyethylene glycol, propylene glycol, triacetin, and a mixture thereof.

7. The water-soluble emulsion composition of claim 1 or 2, wherein the edible oil and fat is selected from soybean oil, corn oil, vegetable oil, high oleic vegetable oil, safflower oil, rice bran oil, palm oil, olive oil, sunflower oil, coconut oil, wheat germ oil, and medium-chain neutral fat (MCT oil).
